(19) Europäisches Patentamt
European Patent Office
Office européen des brevets

(11) EP 0 799 819 B1

(12) **EUROPÄISCHE PATENTSCHRIFT**

(45) Veröffentlichungstag und Bekanntmachung des Hinweises auf die Patenterteilung:
**11.09.2002 Patentblatt 2002/37**

(21) Anmeldenummer: **97102923.6**

(22) Anmeldetag: **22.02.1997**

(51) Int Cl.[7]: **C07C 217/62**, C07C 215/54, C07C 219/28, C07C 211/28, C07C 211/29, C07C 215/64, C07D 333/58, A61K 31/135, A61K 31/235, A61K 31/38

(54) **1-Dimethylaminomethyl-2-phenyl-cycloalk-2-enverbindungen mit analgetischer Wirkung**

1-Dimethylaminomethyl 2-phenyl cycloalk-2-ene derivatives with analgesic activity

Dérivés de 1-diméthylaminométhyl-2-phényl-cycloalc-2-ènes ayant une activité analgésique

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **13.03.1996 DE 19609847**

(43) Veröffentlichungstag der Anmeldung:
**08.10.1997 Patentblatt 1997/41**

(60) Teilanmeldung:
**99119108.1 / 0 983 994**
**99119109.9 / 0 983 995**
**00116745.1 / 1 069 106**

(73) Patentinhaber: **Grünenthal GmbH**
**52078 Aachen (DE)**

(72) Erfinder:
- **Buschmann, Helmut Heinrich, Dr.**
  **52066 Aachen (DE)**
- **Strassburger, Wolfgang Werner Alfred, Prof. Dr.**
  **52146 Würselen (DE)**
- **Friderichs, Elmar Josef Dr.**
  **52223 Stolberg (DE)**
- **Koegel, Babette-Yvonne, Dr.**
  **52379 Langerwehe-Hamisch (DE)**

(56) Entgegenhaltungen:
**EP-A- 0 176 049** **EP-A- 0 693 475**
**US-A- 3 564 100** **US-A- 3 652 589**

- **ARZNEIMITTEL FORSCHUNG. DRUG RESEARCH, Bd. 28, Nr. 1A, 1.Januar 1978, Seiten 107-113, XP000608150 FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN"**
- **FRESENIUS' Z. ANAL. CHEM. (1984), 317(1), 42-52, XP000672648 MAURER, H. ET AL: "Screening procedure for the detection of opioids, other potent analgesics and their metabolites in urine using a computerized gas chromatographic-mass spectrometric technique"**
- **CHEM. PHARM. BULL. (1984), 32(6), 2279-89, XP002031715 KATO, TAKESHI ET AL: "Synthesis and analgesic activity of cyclohexenylmethylamines and related compounds"**
- **CHEMICAL ABSTRACTS, vol. 54, no. 20, 25.Oktober 1960 Columbus, Ohio, US; abstract no. 20963c, I. N. NAZAROV ET AL.: "Synthetic analgesic substances. XXX. Synthesis and study of p-methoxy- and p-chlorophenyldimethylaminopropanols and their esters" XP002031716 & IZVEST. AKAD. NAUK. S.S.S.R., OTDEL. KHIM. NAUK., 1960, Seiten 251-8,**

EP 0 799 819 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft 1-Dimethylaminomethyl-2-phenyl-cycloalk-2-enverbindungen, Verfahren zu deren Herstellung sowie die Verwendung dieser Verbindungen in Arzneimitteln.

Die Behandlung chronischer und nichtchronischer Schmerzzustände hat in der Medizin eine große Bedeutung. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik sowie der Grundlagenforschung zur Nociception in letzter Zeit erschienen sind.

Opioide werden seit vielen Jahren als Analgetika zur Schmerzbehandlung eingesetzt, obwohl sie eine Reihe von Nebenwirkungen, beispielsweise Sucht und Abhängigkeit, Atemdepression, gastro-intestinale Hemmwirkung und Obstipation, hervorrufen. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990).

Tramadolhydrochlorid - (1RS, 2RS)-2-Dimethylaminomethyl-1-(3-methoxy-phenyl)-cyclohexanol, Hydrochlorid - nimmt unter den zentralwirksamen Analgetika eine Sonderstellung ein, da dieser Wirkstoff eine starke Schmerzhemmung ohne die für Opioide bekannten Nebenwirkungen hervorruft (J. Pharmacol. Exptl. Ther. 267, 331 (1993)). Tramadol ist ein Racemat und besteht aus gleichen Mengen an (+)- und (-)-Enantiomer. In vivo bildet der Wirkstoff den Metaboliten O-Desmethyl-tramadol, der gleichfalls als Enantiomerengemisch vorliegt. Untersuchungen haben ergeben, daß sowohl die Enantiomeren von Tramadol als auch die Enantiomeren der Tramadolmetabolite an der analgetischen Wirkung beteiligt sind (J. Pharmacol. Exptl. Ther. 260, 275 (1992)).

Aus Arzneim.-Forschung/Drug Research 28, Heft 1a, 1978, 107-113, sind analgetisch wirksame phenylsubstituierte Dimethyl-aminomethylcyclohexene bekannt, deren Phenylsubstituent in 3-Position mit einer O-$CH_3$-Gruppe substituiert ist.

Die der Erfindung zugrundeliegende Aufgabe bestand in der Entwicklung von analgetisch wirksamen Substanzen, die sich zur Behandlung starker Schmerzen eignen, ohne die für Opioide typischen Nebenwirkungen hervorzurufen.

Darüber hinaus sollten die zu entwickelnden Substanzen nicht die während der Behandlung mit Tramadol in manchen Fällen auftretenden Nebenwirkungen, beispielsweise Übelkeit und Erbrechen, besitzen.

Es wurde gefunden, daß die an die zu entwickelnden Substanzen gestellten Anforderungen von bestimmten 1-Dimethylaminomethyl-2-phenyl-cycloalk-2-enverbindungen erfüllt werden. Diese Substanzen zeichnen sich durch eine ausgeprägte analgetische Wirkung aus, die im Vergleich zu Tramadol deutlich verstärkt ist.

Gegenstand der Erfindung sind dementsprechend 1-Dimethylaminomethyl-2-phenyl-cycloalk-2-enverbindungen der Formel I

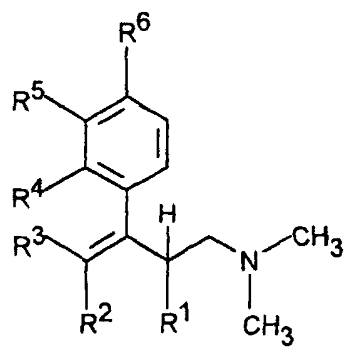

$R^1$ und $R^2$ zusammen-$(CH_2)_{2-4}$-, -$(CH_2)_2$-$CHR^7$ oder -$CH_2$-$CHR^7$-$CH_2$- darstellen, $R^3$ H oder $C_{1-5}$-Alkyl bedeutet, $R^4$ H, OH, $C_{1-4}$-Alkyl, O-$C_{1-4}$-Alkyl, O-Benzyl, $CF_3$, O-$CF_3$, Cl, F oder $OR^8$ bedeutet, $R^5$ H darstellt und $R^6$ H, OH, $C_{1-4}$-Alkyl, O-$C_{1-4}$-Alkyl, O-Benzyl, $CF_3$, O-$CF_3$, Cl, F oder $OR^8$ bedeutet, mit der Maßgabe, daß zwei der Reste $R^4$, $R^5$ oder $R^6$ H sind, $R^7$ $C_{1-8}$-Alkyl, $C_{3-8}$-Cycloalkyl, O-$C_{1-4}$-Alkyl, O-Benzyl, $CF_3$, Cl oder F bedeutet, $R^8$ CO-$C_6H_4$-$R^{11}$, $R^{11}$ OC(O)-$C_{1-3}$-Alkyl in ortho-Stellung oder $CH_2$-N-$(R^{15})_2$ in meta- oder para-Stellung, wobei $R^{15}$ $C_{1-4}$-Alkyl oder beide Reste $R^{15}$ zusammen mit N den 4-Morpolino-Rest bilden, bedeutet, in Form ihrer Basen und/oder Salze von physiologisch verträglichen Säuren, als Enantiomere oder Racemate.

Bevorzugte Verbindungen entsprechen der Formel I mit $R^1$ und $R^2$ zusammen -$(CH_2)_{2-4}$oder -$(CH_2)_2$-$CHR^7$, $R^3$ H oder $C_{1-3}$-Alkyl, $R^4$ H, OH, $CF_3$, Cl, F oder $OR^8$, $R^5$ H und $R^6$ H, OH, O-$C_{1-4}$-Alkyl, O-Benzyl, $CF_3$, Cl, F oder

$OR^8$, mit der Maßgabe, daß zwei der Reste $R^4$, $R^5$ oder $R^6$ H sind und $R^7$ $C_{1-4}$-Alkyl, $CF_3$, Cl oder F bedeutet. Besonders eignen sich Verbindungen der Formel I, in der $R^1$ und $R^2$ zusammen $-(CH_2)_{2-3}$-oder $-(CH_2)_2-CHR^7$ bedeuten, $R^3$ H, $CH_3$ oder $CH_2CH_3$ bedeutet, $R^4$ H oder OH, $R^5$ H und $R^6$ H, OH oder $CF_3$ bedeuten, mit der Maßgabe, daß zwei der Reste $R^4$, $R^5$ oder $R^6$ H sind.

Weiterer Erfindungsgegenstand ist ein Verfahren zur Herstellung von Verbindungen der Formel I, in der $R^1$ und $R^2$ zusammen $-(CH_2)_{2-4}$-, $-(CH_2)_2-CHR^7$ oder $-CH_2-CHR^7-CH_2-$ darstellen, $R^3$ H oder $C_{1-5}$-Alkyl bedeutet, $R^4$ H, $C_{1-4}$-Alkyl, O-$C_{1-4}$-Alkyl, O-Benzyl, $CF_3$, O-$CF_3$, Cl oder F bedeutet, $R^5$ H und $R^6$ H, $C_{1-4}$-Alkyl, O-$C_{1-4}$-Alkyl, O-Benzyl, $CF_3$, O-$CF_3$, Cl oder F bedeutet, mit der Maßgabe, daß zwei der Reste $R^4$, $R^5$ oder $R^6$ H sind, $R^7$ $C_{1-8}$-Alkyl, $C_{3-8}$-Cycloalkyl, O-$C_{1-4}$-Alkyl, O-Benzyl, $CF_3$, Cl oder F bedeutet, welches dadurch gekennzeichnet ist, daß man ein β-Dimethylaminoketon der Formel II

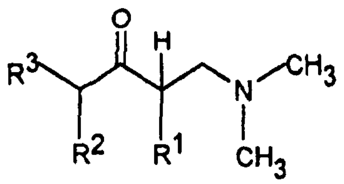

mit einer metallorganischen Verbindung der Formel III

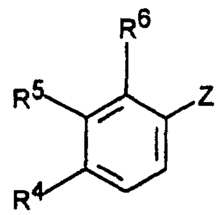

in der Z MgCl, MgBr, MgI oder Li bedeutet, zu einem tertiären Alkohol der Formel IV

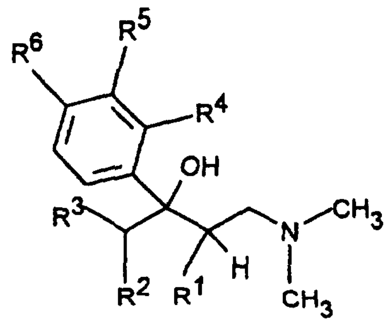

umsetzt, welcher anschließend zu einer Verbindung der Formel I dehydratisiert wird.

Die Reaktion eine β-Dimethylaminketons mit einer Grignard-Verbindung der Formel III, in der Z MgCl, MgBr oder MgJ bedeutet, oder mit einer lithiumorganischen Verbindung der Formel III kann in einem aliphatischen Ether, beispielsweise Diethylether und/oder Tetrahydrofuran, bei Temperaturen zwischen -70° und +60° C durchgeführt werden. Die Umsetzung mit einer Grignard-Verbindung kann mit oder ohne Zusatz eines Mitführreagenzes, vorzugsweise 1,2-Dibromethan, erfolgen. Lithiumorganische Verbindungen der Formel III lassen sich durch Umsetzung einer Verbindung der Formel III, in der Z Cl, Br oder J bedeutet, mit beispielsweise einer n-Butyllithium/Hexan-Lösung durch Halogen/Lithiumaustausch erhalten.

Die erhaltenen tertiären Alkohole der Formel IV lassen sich mit Säuren, insbesondere Ameisensäure oder Salzsäure, bei Temperaturen zwischen 0° und 100° C dehydratisieren.

Weiterer Erfindungsgegenstand ist ein Verfahren zur Herstellung einer Verbindung der Formel I, in der $R^1$ und $R^2$ zusammen $-(CH_2)_{2-4}$-, $-(CH_2)_2-CHR^7$ oder $-CH_2-CHR^7-CH_2-$ bedeuten, $R^3$ H oder $C_{1-5}$-Alkyl bedeutet, einer

der Reste $R^4$ oder $R^6$ OH bedeutet und zwei der Reste $R^4$, $R^5$ oder $R^6$ H sind, $R^7$ $C_{1-8}$-Alkyl, $C_{3-8}$-Cycloalkyl, O-$C_{1-4}$-Alkyl, O-Benzyl, $CF_3$, Cl oder F bedeutet, welches dadurch gekennzeichnet ist, daß man eine Verbindung der Formel I, in der einer der Reste $R^4$ oder $R^6$ O-$CH_3$ bedeutet und zwei der Reste $R^4$, $R^5$ oder $R^6$ H sind, mit Diisobutylaluminiumhydrid umsetzt oder eine Verbindung der Formel I, in der einer der Reste $R^4$ oder $R^6$ O-Benzyl bedeutet und zwei der Reste $R^4$, $R^5$ oder $R^6$ H sind, reduktiv debenzyliert.

Die Umsetzung einer 1-Dimethylaminomethyl-2-phenyl-cycloalk-2-enverbindung mit Diisobutylaluminiumhydrid wird üblicherweise in einem aromatischen Kohlenwasserstoff, beispielsweise Toluol, bei einer Temperatur zwischen 60° und 130° C durchgeführt (Synthesis 1975, 617; DE 24 09 990, DE 24 09 991; Chem. Abstr. 84, 59862 (1974)).

Die reduktive Debenzylierung einer erfindungsgemäßen Verbindung der Formel I, in der einer der Reste $R^4$ oder $R^6$ O-Benzyl bedeutet, läßt sich in Gegenwart von Platin oder Paladium auf einem Trägermaterial, beispielsweise Aktivkohle, in Gegenwart von Wasserstoff in einem Lösungsmittel, beispielsweise Essigsäure oder $C_{1-4}$-Alkylalkohol, bei Drücken zwischen 1 und 100 bar und Temperaturen zwischen 20° und 100° C durchführen.

Verbindungen der allgemeinen Formel I, in der einer oder mehrere der Aromatensubstituenten $R^4$ und $R^6$ $OR^8$ bedeuten und $OR^8$ eine Carboxylat-Gruppe darstellt, lassen sich durch Umsetzung einer entsprechenden Verbindung der Formel I, in der $R^4$ und/oder $R^6$ eine OH-Gruppe bedeuten, in Form eines Alkalisalzes mit einem Carbonsäurechlorid erhalten. Diese Umsetzungen werden üblicherweise in einem Lösungsmittel, beispielsweise Toluol, Dichlormethan, Diethylether und/oder Tetrahydrofuran bei Temperaturen zwischen -15° und +110° C durchgeführt (Drugs of the Future 16, 443 (1991); J. Med. Chem. 30, 2008 (1989) und 32, 2503 (1989); J. Org. Chem. 43, 4797 (1978); Tetrahedron Lett. 1977, 1571; J. Pharm. Sci. 57, 774 (1968)).

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in wäßriger Lösung.

Die erfindungsgemäßen Verbindungen haben eine ausgeprägte analgetische Wirkung und sind toxikologisch unbedenklich. Sie eignen sich daher als pharmazeutische Wirkstoffe. Dementsprechend ist Erfindungsgegenstand auch die Verwendung einer 1-Dimethylaminomethyl-2-phenyl-cycloalk-2-enverbindung der Formel I als Wirkstoff in Arzneimitteln, vorzugsweise als Wirkstoff in Schmerzmitteln.

Erfindungsgemäße Arzneimittel enthalten neben mindestens einer Verbindung der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, intravenös, intraperitoneal, intradermal, intramuskulär, intranasal, buccal oder örtlich, zum Beispiel auf Infektionen an der Haut, an den Schleimhäuten oder an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot in gelöster Form oder in einem Pflaster, gegebenenfalls unter Zusatz von die Hauptpenetration fördernden Mitteln, sind Beispiele für geeignete perkutane Zubereitungsformen. Aus oral oder perkutan anwendbaren Zubereitungsformen können die erfindungsgemäßen Verbindungen verzögert freigesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 10 bis 500 mg pro kg wenigstens einer Verbindung der Formel I appliziert.

**Beispiele**

**Herstellung erfindungsgemäßer Verbindungen**

Die Angabe Ether bedeutet Diethylether.

Als stationäre Phase für die Säulenchromatographie wurde Kieselgel 60 (0,040 - 0,063 mmm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Racemattrennungen wurden auf einer Chiracel OD Säule der Firma Daicel Chemical Industries, LTD durchgeführt.

Die Mischungsverhältnisse der Laufmittel für alle chromatographischen Untersuchungen sind in Volumen/Volumen angegeben.

**Beispiel 1 (nicht Gegenstand der Ansprüche)**

(Z)-(RS)-[3-(3-Methoxy-phenyl)-2-methyl-pent-3-enyl]-dimethylamin, Hydrochlorid (1)

1. Stufe

(2RS, 3RS)-1-Dimethylamino-3-(3-methoxy-phenyl)-2-methyl-pentan-3-ol, Hydrochlorid (2)

27,0 g (1,11 mol) Magnesiumspäne wurden in 150 ml Tetrahydrofuran gerührt und 207,6 g (1,11 mol) 1-Brom-3-methoxy-benzol, gelöst in 400 ml Tetrahydrofuran, zugetropft. Es wurde eine Stunde unter Rückfluß erhitzt und anschließend auf eine Temperatur zwischen 5° C und 10° C abgekühlt. Bei dieser Temperatur wurden 128,30 g (0,89 mol) (RS)-1-Dimethylamino-2-methyl-pentan-3-on, gelöst in 400 ml Tetrahydrofuran, zugetropft. Das Reaktionsgemisch wurde stehen gelassen und anschließend erneut auf eine Temperatur zwischen 5° C und 10° C abgekühlt. Nach Zugabe von 300 ml 20 gew.%iger Ammoniumchloridlösung wurde mit 400 ml Ether verdünnt. Nach Phasentrennung wurde zweimal mit Ether extrahiert, über Natriumsulfat getrocknet und das Lösungsmittel destillativ entfernt. Der erhaltene Rückstand wurde in 3,2 l 2-Butanon aufgenommen und mit 120,60 g (1,11 mol) Trimethylchlorsilan und 20 ml Wasser versetzt. Es wurden 121,5 g Hydrochlorid (2) (38 % der Theorie) mit einem Schmelzpunkt von 198 - 199° C erhalten.

2. Stufe:

(Z)-(RS)-[3-(3-Methoxy-phenyl)-2-methyl-pent-3-enyl]-dimethylamin, Hydrochlorid (1)

200 g (0,69 mol) Hydrochlorid (2) wurden in einem Liter konzentrierter Salzsäure gelöst und bei Raumtemperatur stehengelassen. Die Salzsäure wurde im Vakuum destillativ entfernt. Der Rückstand wurde in 1 l Eiswasser gelöst und mit 10 molarer Natronlauge ein pH-Wert von 13 eingestellt. Nach Extraktion mit Ether, Trocknen der organischen Phase und destillativer Entfernung des Lösungsmittels wurden 162 g Rohprodukt erhalten, das durch umkristallisieren gereinigt wurde. Es wurden 79 g (42 % der Theorie) Hydrochlorid (1) mit einem Schmelzpunkt von 169 - 170° C erhalten.

**Beispiel 2 (nicht gegenstand der Ansprüche)**

(Z)-(RS)-3-[1-(2-Dimethylamino-1-methyl-ethyl)-propenyl]-phenol, Hydrochlorid (3)

Zu 1,6 l 20-gew.%iger Diisobutylaluminiumhydrid-Lösung in Toluol wurden bei Raumtemperatur 182 g (Z)-(RS)-[3-(3-Methoxy-phenyl)-2-methyl-pent-3-enyl]-dimethylamin, gelöst in 360 ml Toluol, getropft. Anschließend wurde 11 Stunden unter Rückfluß erhitzt. Nach Abkühlen auf 0° C wurden 450 ml Ethanol unter Kühlung zugetropft. Anschließend wurde 15 Minuten gerührt und mit 1 l Toluol verdünnt. Danach wurden 450 ml eines Ethanol/Wasser-Gemisches (1 : 1) unter Kühlung zugetropft. Nach einstündigem Rühren bei Raumtemperatur wurde das ausgefallene Aluminiumhydroxid abgesaugt und aus der organischen Phase Lösungsmittel destillativ entfernt. Es wurden 167 g (97,6 % der Theorie) Rohbase erhalten, die in 1,67 l Aceto gelöst und mit 65 ml konzentrierter Salzsäure versetzt wurde. Es kristallisierten 152 g (76 % der Theorie) Hydrochlorid (3) mit einem Schmelzpunkt von 161 - 162° C aus.

**Beispiel 3 (nicht gegenstand der Ansprüche)**

Enantiomere von (3):

(+)-(Z)-(S)-3-[1-(2-Dimethylamino-1-methyl-ethyl)-propenyl]-phenol, Hydrochlorid (+3) und

-(Z)-(R)-3-[1-(2-Dimethylamino-1-methyl-ethyl)-propenyl]-phenol, Hydrochlorid (-3)

Aus dem nach Beispiel 2 erhaltenen Hydrochlorid (3) wurde mit Dichlormethan/wäßriger Natriumhydrogencarbonatlösung die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 166 - 167° C isoliert.

(+3): Ausbeute: 42 % der Theorie $[\alpha]_D^{RT}$ = +3,6° (c = 1,04; Methanol)

(-3): Ausbeute: 44 % der Theorie $[\alpha]_D^{RT}$ = -3,6° (c = 1,04; Methanol)

**Beispiel 4 (nicht gegenstand der Ansprüche)**

(Z)-(RS)-2-Acetoxy-benzoesäure-3-[1-(2-dimethylamino-1-methyl-ethyl)-propenyl]-phenylester, Hydrochlorid (4)

Aus dem Hydrochlorid (3), hergestellt nach Beispiel 2, wurde mit Dichlormethan/wäßriger Natriumhydrogencarbonat-Lösung die Base freigesetzt und nach dem Trocknen der Lösung das Dichlormethan destillativ entfernt. 0,67 g (3,0 mmol) der erhaltenen Base wurden in 7 ml trockenem Dichlormethan gelöst und bei Raumtemperatur mit 0,6 g (3,24 mmol) 2-Acetyl-benzoyl-chlorid, gelöst in 3 ml trockenem Dichlormethan, versetzt. Nach 20-stündigem Rühren bei Raumtemperatur wurde das Reaktionsgemisch mit 20 ml Natriumhydrogencarbonatlösung versetzt und die wäßrige Phase zweimal mit 10 ml Dichlormethan extrahiert. Die organischen Phasen wurden vereinigt und über Natriumsulfat getrocknet. Nach destillativer Entfernung des Lösungsmittels wurden 1,1 g Rohgemisch gewonnen, das auf eine Säule, gefüllt mit Kieselgel, gegeben wurde. Die Elution mit Ether ergab 0,68 g Base, aus der mit Trimethylchlorsilan/Wasser in Ether 0,68 g (54 % der Theorie) Hydrochlorid (4) mit einem Schmelzpunkt von 86 - 88° C erhalten wurden.

**Beispiel 5 (nicht gegenstand der Ansprüche)**

(E)-(RS)-[3-(3-Methoxy-phenyl)-2-methyl-pent-3-enyl)-dimethylamin, Hydrochlorid (5)

75 g (0,26 mol) (2RS,3RS)-1-Dimethylamino-3-(3-methoxyphenyl)-2-methyl-pentan-3-ol, Hydrochlorid (1) aus Beispiel 1 (Stufe 1) wurden in einem Liter konzentrierter Ameisensäure gelöst und zwei Stunden unter Rückfluß erhitzt. Anschließend wurde die Ameisensäure im Wasserstrahlvakuum abdestilliert, der Rückstand in Eiswasser aufgenommen und mit Natronlauge/Ether versetzt. Nach Trocknung der organischen Phase und destillativer Entfernung des Lösungsmittels wurden 60 g (98 % der Theorie) Rohbase erhalten ((Z)-Isomer (2) : (E)-Isomer (5) = 6 : 4). Die Rohbase wurde auf eine Säule, gefüllt mit Kieselgel, gegeben. Die Elution mit Diisopropylether/Methanol = 7 : 1 ergab 20 g Base, aus der mit Trimethylchlorsilan/Wasser in 2-Butanon 18,4 g (26 % der Theorie) Hydrochlorid (5) mit einem Schmelzpunkt von 139 - 140 °C erhalten wurden.

**Beispiel 6 (nicht gegenstand der Ansprüche)**

(E)-(RS)-3-[1-(2-Dimethylamino-1-methyl-ethyl)-propenyl]-phenol, Hydrochlorid (6)

Aus (5), hergestellt nach Beispiel 5, wurde mit Dichlormethan/Natronlauge die Base freigesetzt und nach dem Trocknen der Lösung Dichlormethan destillativ entfernt. Aus der erhaltenen Base wurde unter den in Beispiel 2 angegebenen Bedingungen das Hydrochlorid (6) in einer Ausbeute von 73 % der Theorie und einem Schmelzpunkt von 80° C erhalten.

**Beispiel 7 (nicht gegenstand der Ansprüche)**

Enantiomere von (6):

(+)-(E)-(R)-3-[1-(2-Dimethylamino-1-methyl-ethyl)-propenyl]-phenol, Hydrochlorid (+6) und

(-)-(E)-(S)-3-[1-(2-Dimethylamino-1-methyl-ethyl)-propenyl]-phenol, Hydrochlorid (-6)

Aus nach Beispiel 6 erhaltenem Hydrochlorid (6) wurde mit Dichlormethan/wäßriger Natriumhydrogencarbonatlösung die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 154 - 155° C isoliert.

(+6): Ausbeute: 42 % der Theorie $[\alpha]_D^{RT}$ = +36,3° (c = 0,96; Methanol)

(-6): Ausbeute: 44 % % der Theorie $[\alpha]_D^{RT}$ = -33,7° (c = 1,07; Methanol)

**Beispiel 8 (nicht gegenstand der Ansprüche)**

(Z)-(RS)-4-[1-(2-Dimethylamino-1-methyl-ethyl)-propenyl]-phenol, Hydrochlorid (7)

1. Stufe:

(Z)-(RS)-[3-(4-Methoxy-phenyl)-2-methyl-pent-3-enyl]-dimethylamin (8)

Ausgehend von (RS)-1-Dimethylamino-2-methyl-pentan-3-on und 1-Brom-4-methoxy-benzol wurde unter den in Beispiel 1 (1. Stufe) angegebenen Bedingungen (2RS, 3RS)-1-Dimethylamino-3- (4-methoxy-phenyl)-2-methyl-pentan-3-ol, Hydrochlorid in einer Ausbeute von 44 % und einem Schmelzpunkt von 188 - 189° C erhalten, das unter den in Beispiel 1 (2. Stufe) angegebenen Bedingungen mit konzentrierter Salzsäure in (Z)-(RS)-[3-(4-Methoxy-phenyl)-2-methyl-pent-3-enyl]-dimethylamin (8) überführt wurde. Verbindung (8) wurde als hellgelbes Öl in einer Ausbeute von 46 % erhalten.

2. Stufe:

(Z)-(RS)-4-[1-(2-Dimethylamino-1-methyl-ethyl)-propenyl]-phenol, Hydrochlorid (7)

Aus der nach Stufe 1 erhaltenen Base wurde unter den in Beispiel 2 angegebenen Bedingungen das Hydrochlorid (7) in einer Ausbeute von 79 % der Theorie und einem Schmelzpunkt von 203° C erhalten.

**Beispiel 9 (nicht gegenstand der Ansprüche)**

(Z)-(RS)-Dimethyl-(2-methyl-3-m-tolyl-pent-3-enyl)amin, Hydrochlorid (9)

Ausgehend von (RS)-1-Dimethylamino-2-methyl-pentan-3-on und 3-Brom-toluol wurde unter den in Beispiel 1 (1. Stufe) angegebenen Bedingungen (2RS, 3RS)-1-Dimethylamin-2-methyl-3-(m-tolyl)-pentan-3-ol, Hydrochlorid in einer Ausbeute von 24 % und einem Schmelzpunkt von 154 - 155° C erhalten, das unter den in Beispiel 1 (2. Stufe) angegebenen Bedingungen mit konzentrierter Salzsäure in (Z)-(RS)-Dimethyl-(2-methyl-3-m-tolylpent-3-enyl)-amin, Hydrochlorid (9) überführt wurde. Verbindung (9) wurde in einer Ausbeute von 36 % (bezogen auf den eingesetzten Alkohol) mit einem Schmelzpunkt von 172° C erhalten.

**Beispiel 10 (nicht gegenstand der Ansprüche)**

(E)-(RS)-Dimethyl-(2-methyl-3-m-tolyl-pent-3-enyl)amin, Hydrochlorid (10)

Ausgehend von (2RS, 3RS)-1-Dimethylamino-2-methyl-3-(m-tolyl)-pentan-3-ol, Hydrochlorid, welches nach Beispiel 9 hergestellt wurde, wurde unter den in Beispiel 5 angegebenen Bedingungen das Hydrochlorid (10) in einer Ausbeute von 36 % mit einem Schmelzpunkt von 153° C erhalten.

**Beispiel 11 (nicht gegenstand der Ansprüche)**

(Z)-(RS)-[3-(3-Difluoromethyl-phenyl)-2-methyl-pent-3-enyl]-dimethylamin, Hydrochlorid (11)

1. Stufe:

(2RS,3RS)-3-(3-Difluormethyl-phenyl)-1-dimethylamino-2-methyl-pentan-3-ol, Hydrochlorid (12)

7,0 g (34 mmol) 1-Bromo-3-difluormethyl-benzol, hergestellt aus 3-Brombenzaldehyd und Diethylaminoschwefeltrifluorid gemäß Org. React. 35, 513 (1988), wurden in 110 ml trockenem Tetrahydrofuran gelöst und auf -75° C gekühlt. Nach Zugabe von 34 mmol 1,6-molarer n-Butyllithiümlösung in Hexan wurde eine Stunde bei -75° C gerührt. Anschließend wurden 4,8 g (34 mmol) (2RS)-1-Dimethylamino-2-methyl-pentan-3-on, gelöst in 15 ml trokkenem Tetrahydrofuran, zugetropft. Innerhalb von 2,5 Stunden wurde das Reaktionsgemisch auf Raumtemperatur erwärmt.

Zur Aufarbeitung wurden unter Eisbadkühlung 65 ml 5 %-ige Salzsäure zugetropft, so daß die Innentemperatur 15° C nicht überstieg. Nach Phasentrennung wurde die organische Phase mit 40 ml 5 %-iger Salzsäure extrahiert. Die vereinigten wäßrigen Phasen wurden zweimal mit 50 ml Ether gewaschen. Zur Freisetzung der Base wurde mit

konzentrierter Natronlauge versetzt und mit Dichlormethan extrahiert. Auf diese Weise wurden 7,8 g Rohprodukt erhalten , das auf eine Säule, gefüllt mit Kieselgel, gegeben wurde. Die Elution mit Essigsäureethylester/Methanol = 1 : 1 ergab 4,89 g Base, aus der mit Trimethylchlorsilan/Wasser in 2-Butanon 4,6 g (44 % der Theorie) Hydrochlorid (12) mit einem Schmelzpunkt von 194 - 195° C erhalten wurde.

2. Stufe:

(Z)-(RS)-[3-(3-Difluormethyl-phenyl)-2-methyl-pent-3-enyl]-dimethyl-amin, Hydrochlorid (11)

10 g (32 mmol) (2RS, 3RS)-3-(3-Difluormethyl-phenyl)-1-dimethylamino-2-methyl-pentan-3-ol, Hydrochlorid (12) aus Stufe 1 wurden in 150 ml konzentrierter Ameisensäure gelöst und zwei Stunden unter Rückfluß erhitzt. Anschließend wurde die Ameisensäure im Wasserstrahlvakuum abdestilliert, der Rückstand in Eiswasser aufgenommen und mit Natronlauge/Ether versetzt. Nach Trocknung der organischen Phase und destillativer Entfernung des Lösungsmittels wurden 9,1 g (97 % der Theorie) Rohbase erhalten, die auf eine Säule, gefüllt mit Kieselgel, gegeben wurden. Die Elution mit Diisopropylether/Methanol = 7 : 1 ergab 3,0 g Base, aus der mit Trimethylchlorsilan/Wasser in 2-Butanon 2,3 g (24 % der Theorie) Hydrochlorid (11) mit einem Schmelzpunkt von 160 - 161° C erhalten wurden.

**Beispiel 12 (nicht gegenstand der Ansprüche)**

(Z)-(RS)-6-[1-(2-Dimethylamino-1-methyl-ethyl)-propenyl]-naphth-2-ol, Hydrochlorid (13)

Aus (1RS, 2RS)-6-(3-Dimethylamino-1-ethyl-1-hydroxy-2-methyl-propyl)-naphth-2-ol, Hydrochlorid, hergestellt gemäß Chirality 6, 389 (1994), wurde unter den in Beispiel 1 (2. Stufe) angegebenen Bedingungen das Hydrochlorid (13) in 39 %-iger Ausbeute mit einem Schmelzpunkt von 207 - 208° C erhalten.

**Beispiel 13 (nicht gegenstand der Ansprüche)**

(E)-(RS)-[3-(3-Methoxy-phenyl)-2-methyl-hex-3-enyl]-dimethylamin, Hydrochlorid (14) und

(Z)-(RS)-[3-(3-Methoxy-phenyl)-2-methyl-hex-3-enyl]-dimethylamin, Hydrochlorid (15)

Ausgehend von (2RS)-3-Dimethylamino-1-(3-methoxy-phenyl)-2-methyl-propan-1-on und 1-Brom-propan wurde unter den in Beispiel 1 (1. Stufe) angegebenen Bedingungen unter Verwendung von Ether als Lösungsmittel das (2RS, 3SR)-1-Dimethylamino-3-(3-methoxy-phenyl)-2-methyl-hexan-3-ol, Hydrochlorid (16) in einer Ausbeute von 81 % mit einem Schmelzpunkt von 131 - 132° C erhalten. 30 g (0,1 mol) der Verbindung (16) wurden gemäß Beispiel 5 mit 450 ml konzentrierter Ameisensäure umgesetzt. Die auf diese Weise erhaltene Rohbase (28 g), bestehend aus einem (Z)/(E)(Z)/(E)-Isomerengemisch wurde auf eine Säule, gefüllt mit Kieselgel, gegeben. Die Elution mit Diisopropylether/Methanol = 7 : 1 ergab 7 g Base der (E)-Verbindung (14) und 17 g Base der (Z)-Verbindung (15). Die Basen wurden mit Trimethylchlorsilan/ Wasser in 2-Butanon in die Hydrochloride überführt.

(14) Ausbeute: 5,9 g (21 % der Theorie) Schmelzpunkt: 154° C

(15) Ausbeute: 15,8 g (56 % der Theorie) Schmelzpunkt: 110 - 112° C

**Beispiel 14 (nicht gegenstand der Ansprüche)**

(E)-(RS)-3-[1-(2-Dimethylamino-1-methyl-ethyl)-but-1-enyl]-phenol, Hydrochlorid (17)

Aus (14), hergestellt nach Beispiel 13, wurde mit Dichlormethan/Natronlauge die Base freigesetzt und nach dem Trocknen der Lösung Dichlormethan destillativ entfernt. Aus der so erhaltenen Base wurde unter den in Beispiel 2 angegebenen Bedingungen das Hydrochlorid (17) in einer Ausbeute von 86 % der Theorie und einem Schmelzpunkt von 214° C erhalten.

**Beispiel 15 (nicht gegenstand der Ansprüche)**

(Z)-(RS)-3-[1-(2-Dimethylamino-1-methyl-ethyl)-but-1-enyl]-phenol, Hydrochlorid (18)

Aus (15), hergestellt nach Beispiel 13, wurde mit Dichlormethan/Natronlauge die Base freigesetzt und nach

dem Trocknen der Lösung Dichlormethan destillativ entfernt. Aus der so erhaltenen Base wurde unter den in Beispiel 2 angegebenen Bedingungen das Hydrochlorid (18) in einer Ausbeute von 86 % der Theorie und einem Schmelzpunkt von 120 - 121° C erhalten.

**Beispiel 16 (nicht gegenstand der Ansprüche)**

(RS)-[3-(3-Methoxy-phenyl)-2-propyl-but-3-enyl]-dimethylamin, Hydrochlorid (19)

Ausgehend von (RS)-2-Dimethylaminomethyl-1-(3-methoxyphenyl)-pentan-1-on und Methyliodid wurde unter den in Beispiel 1 (1. Stufe) angegebenen Bedingungen unter Verwendung von Ether als Lösungsmittel (2RS, 3SR)-3-Dimethylaminomethyl-2-(3-methoxy-phenyl)-hexan-2-ol, Hydrochlorid (20) in einer Ausbeute von 76 % mit einem Schmelzpunkt von 137 - 138° C erhalten. 30 g (0,1 mol) der Verbindung (20) wurden gemäß Beispiel 5 mit 300 ml konzentrierter Ameisensäure umgesetzt. Die erhaltene Rohbase wurde auf eine Säule, gefüllt mit Kieselgel, gegeben. Die Elution mit Diisopropylether/Methanol = 7 : 1 ergab 24 g Base, aus der mit Trimethylchlorsilan/Wasser in 2-Butanon 23,1 g (74 % der Theorie) Hydrochlorid (19) mit einem Schmelzpunkt von 120 - 121° C erhalten wurden.

**Beispiel 17 (nicht gegenstand der Ansprüche)**

(RS)-3-[1-(2-Dimethylamino-1-methyl-ethyl]-vinyl]-phenol, Hydrochlorid (21)

1. Stufe:

(1RS, 2SR)-3-(3-Dimethylamino-1-hydroxy-1,2-dimethylpropyl)-phenol, Hydrochlorid (22)

Ausgehend von (RS)-3-Dimethylamino-1-(3-methoxy-phenyl)-2-methyl-propan-1-on und Methyliodid wurde unter den in Beispiel 1 (1. Stufe) angegebenen Bedingungen unter Verwendung von Ether als Lösungsmittel (2RS, 3SR)-4-Dimethylamino-2-(3-methoxy-phenyl)-3-methyl-butan-2-ol, Hydrochlorid (23) in einer Ausbeute von 46 % mit einem Schmelzpunkt von 178 - 179° C erhalten. Aus (23) wurde mit Dichlormethan/Natronlauge die Base freigesetzt. Nach dem Trocknen der Lösung wurde Dichlormethan im Vakuum abdestilliert. 23,7 g (0,1 mol) der Base wurden gemäß Beispiel 2 2 mit Diisobutylaluminiumhydrid umgesetzt. Auf diese Weise konnten 18,5 g (71 % der Theorie) Hydrochlorid (22) mit einem Schmelzpunkt von 183 - 184° C erhalten werden.

2. Stufe:

(RS)-3-[1-(2-Dimethylamino-1-methyl-ethyl]-vinyl]-phenol, Hydrochlorid (21)

10 g (37 mmol) Hydrochlorid (22) aus Stufe 1 wurden in 150 ml konzentrierter Ameisensäure gelöst und zwei Stunden unter Rückfluß erhitzt. Anschließend wurde die Ameisensäure im Wasserstrahlvakuum abdestilliert, der Rückstand in Eiswasser aufgenommen und mit Natronlauge/Ether versetzt. Nach Trocknung der organischen Phase und destillativer Entfernung des Lösungsmittels wurden 9,1 g Rohbase erhalten, aus der mit konzentrierter Salzsäure in Aceton 7,5 g (83 % der Theorie) Hydrochlorid (21) mit einem Schmelzpunkt von 228 - 230° C erhalten wurden.

**Beispiel 18 (nicht gegenstand der Ansprüche)**

(RS)-3-[1-(2-Dimethylamino-1-methyl-ethyl)-2-methylpropenyl]-phenol, Hydrochlorid (24)

1. Stufe:

(RS)-[3-(3-Methoxy-phenyl)-2,4-dimethyl-pent-3-enyl]-dimethylamin (25) Ausgehend von (RS)-1-Dimethylamino-2,4-dimethyl-pentan-3-on und 1-Brom-3-methoxy-benzol wurde unter den in Beispiel 1 (1. Stufe) angegebenen Bedingungen (2RS, 3RS)-1-Dimethylamino-3-(3-methoxy-phenyl)-2,4-dimethylpentan-3-ol, Hydrochlorid (26) in einer Ausbeute von 44 % mit einem Schmelzpunkt von 180 - 181° C erhalten. 30 g (0,1 mol) der Verbindung (26) wurden gemäß Beispiel 5 mit 450 ml konzentrierter Ameisensäure umgesetzt. Die erhaltene Rohbase wurde auf eine Säule,

gefüllt mit Kieselgel, gegeben. Die Elution mit Diisopropylether/Methanol = 7 : 1 ergab 19 g Base (77 % der Theorie) als hellgelbes viskoses Öl.

2. Stufe:

(RS)-3-[1-(2-Dimethylamino-1-methyl-ethyl)-2-methylpropenyl]-phenol, Hydrochlorid (24)

Aus der nach Stufe 1 erhaltenen Base wurde unter den in Beispiel 2 angegebenen Bedingungen das Hydrochlorid (24) in einer Ausbeute von 84 % der Theorie und einem Schmelzpunkt von 176 - 177° C erhalten.

**Beispiel 19**

(RS)-Dimethyl-[2-(4-trifluormethyl-phenyl)-cyclopent-2-enylmethyl]-amin, Hydrochlorid (27)

(RS)-2-Dimethylaminomethyl-cyclopentanon und 1-Brom-4-trifluormethyl-benzol wurden unter den in Beispiel 1 (1. Stufe) angegebenen Bedingungen umgesetzt. 30 g des erhaltenen Rohproduktes wurden auf eine Säule, gefüllt mit Kieselgel, gegeben. Die Elution mit Essigsäureethylester/Methanol = 5 : 1 ergab 11,6 g Base, die mit Trimethylchlorsilan/Wasser in 2-Butanon in 12,0 g (21 % der Theorie) (1RS,2RS)-2-Dimethylaminomethyl-1-(4-trifluormethyl-phenyl)-cyclopentanol, Hydrochlorid (28) mit einem Schmelzpunkt von 213 - 214° C überführt wurden. 32,4 g (0,1 mol) des Hydrochlorids (28) wurden gemäß Beispiel 5 mit 450 ml konzentrierter Ameisensäure umgesetzt. Die erhaltene Rohbase wurde auf eine Säule, gefüllt mit Kieselgel, gegeben. Die Elution mit Diisopropylether/Methanol = 7 : 1 ergab 9,6 g Base, die mit Trimethylchlorsilan/Wasser in 2-Butanon in 8,9 g (29 % der Theorie Hydrochlorid (27) mit einem Schmelzpunkt von 219 - 220° C überführt wurden.

**Beispiel 20**

Enantiomere von (27):

(+)-(S)-Dimethyl-[2-(4-trifluormethyl-phenyl)-cyclopent-2-enylmethyl]-amin, Hydrochlorid (+27) und

(-)-(R)-Dimethyl-[2-(4-trifluormethyl-phenyl)-cyclopent-2-enylmethyl]-amin, Hydrochlorid (-27)

Aus (27) wurde mit Dichlormethan/Natronlauge die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 244 - 246° C hergestellt.

(+27): Ausbeute: 42 % der Theorie $[\alpha]_D^{RT}$ = +33,8° (c = 1,00; Methanol)

(-27): Ausbeute: 44 % der Theorie $[\alpha]_D^{RT}$ = -34,3° (c = 1,06; Methanol)

**Beispiel 21**

(RS)-2-(6-Dimethylaminomethyl-cyclohex-1-enyl)-phenol, Hydrochlorid (29)

Ausgehend von (RS)-2-Dimethylaminomethyl-cyclohexanon und 1-Brom-2-methoxy-benzol wurde unter den in Beispiel 1 (1. Stufe) angegebenen Bedingungen unter Verwendung von Ether als Lösungsmittel (1RS,2RS)-2-Dimethylaminomethyl-1-(2-methoxy-phenyl)-cyclohexanol, Hydrochlorid (30) in einer Ausbeute von 47 % erhalten. Aus (30) wurde mit Dichlormethan/Natronlauge die Base freigesetzt. Nach dem Trocknen der Lösung wurde Dichlormethan im Vakuum abdestilliert. 30,0 g (0,1 mol) der Base wurden gemäß Beispiel 2 mit Diisobutylaluminiumhydrid umgesetzt. Es wurden 22,7 g (78 % der Theorie) (1RS,2RS)-2-(2-Dimethylaminomethyl-1-hydroxy-cyclo-hexyl)-phenol, Hydrochlorid (31) mit einem Schmelzpunkt von 168 - 170° C erhalten. 28,6 g (0,1 mol) der Verbindung (31) wurden gemäß Beispiel 5 mit 450 ml konzentrierter Ameisensäure umgesetzt. Die erhaltene Rohbase wurde auf eine Säule, gefüllt mit Kieselgel, gegeben und mit Diisopropylether/Methanol = 7 : 1 eluiert. Es wurden 21 g Base gewonnen, aus der mit konzentrierter Salzsäure in Aceton 18,6 g (69 % der Theorie) Hydrochlorid (29) mit einem Schmelzpunkt von 168° C erhalten wurde.

**Beispiel 22**

Enantiomere von (29):

(-)-(R)-2-(6-Dimethylaminomethyl-cyclohex-1-enyl)-phenol, Hydrochlorid (-29) und

(+)-(S)-2-(6-Dimethylaminomethyl-cyclohex-1-enyl)-phenol, Hydrochlorid (+29)

Aus (29) wurde mit Dichlormethan/wäßriger Natriumhydrogencarbonatlösung die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 271 - 272° C isoliert.

(+29): Ausbeute: 43 % der Theorie $[\alpha]_D^{RT}$ = +24,1° (c = 0,96; Methanol)

(-29): Ausbeute: 44 % der Theorie $[\alpha]_D^{RT}$ = -23,5° (c = 0,94; Methanol)

**Beispiel 23**

(RS)-Dimethyl-[2-(4-trifluoromethyl-phenyl)-cyclohex-2-enylmethyl]-amin, Hydrochlorid (32)

(RS)-2-Dimethylaminomethyl-cyclohexanon und 1-Brom-4-trifluormethyl-benzol wurden unter den in Beispiel 1 (1. Stufe) angegebenen Bedingungen umgesetzt. 30 g des Rohproduktes wurden auf eine Säule, gefüllt mit Kieselgel, gegeben. Die Elution mit Essigsäureethylester/Methanol = 5 : 1 ergab 18,9 g Base, die mit Trimethylchlorsilan/Wasser in 2-Butanon in 16,4 g (37 % der Theorie) (1RS,2RS)-2-Dimethylaminomethyl-1-(4-trifluormethyl-phenyl)-cyclohexanol, Hydrochlorid (33) mit einem Schmelzpunkt von 234° C überführt wurden. 33,7 g (0,1 mol) des Hydrochlorids (33) wurden gemäß Beispiel 5 mit 450 ml konzentrierter Ameisensäure umgesetzt. Die erhaltene Rohbase wurde auf eine Säule, gefüllt mit Kieselgel, gegeben und mit Diisopropylether/ Methanol = 7 : 1 eluiert. Es wurden 12,3 g Base erhalten, die mit Trimethylchlorsilan/Wasser in 2-Butanon in 10,4 g (32,5 % der Theorie Hydrochlorid (32) mit einem Schmelzpunkt von 205 - 206 °C überführt wurden.

**Beispiel 24 (nicht gegenstand der Ansprüche)**

(RS)-Dimethyl-[2-(2-methyl-benzo[b]thiophen-4-yl)-cyclohex-2-enylmethyl]-amin, Hydrochlorid (34)

(RS)-2-Dimethylaminomethyl-cyclohexanon und 4-Brom-2-methyl-benzo[b]thiophen wurden unter den in Beispiel 1 (1. Stufe) angegebenen Bedingungen unter Verwendung von Ether als Lösungsmittel und 1,2-Dibromethan als Mitführreagenz umgesetzt. 25 g des Rohproduktes wurden auf eine Säule, gefüllt mit Kieselgel, gegeben. Die Elution mit Essigsäureethylester/Methanol = 1 : 1 ergab 12,6 g Base, die mit Trimethylchlorsilan/Wasser /Wasser in 2-Butanon in 10,4 g (29 % der Theorie) (1RS,2RS)-2-Dimethylaminomethyl-1-(2-methyl-benzo[b]thiophen-4-yl)-cyclohexanol, Hydrochlorid (35) mit einem Schmelzpunkt von 204° C überführt wurden. 34,0 g (0,1 mol) des Hydrochlorids (35) wurden gemäß Beispiel 5 mit 450 ml konzentrierter Ameisensäure umgesetzt. Die auf diese Weise erhaltene Rohbase (28,4 g) wurde auf eine Säule, gefüllt mit Kieselgel, gegeben. Die Elution mit Ether ergab 17,5 g Base, die mit Trimethylchlorsilan/Wasser in 2-Butanon in 15,2 g (54,8 % der Theorie) Hydrochlorid (34) mit einem Schmelzpunkt von 179 - 182° C überführt wurden.

**Beispiel 25 (nicht gegenstand der Ansprüche)**

(-)-(3S,6R)-3-(6-Dimethylaminomethyl-3-methyl-cyclohex-1-enyl)-phenol, Hydrochlorid (-36) und

(+) - (3R,6S)-3-(6-Dimethylaminomethyl-3-methyl-cyclohex-1-enyl)-phenol, Hydrochlorid (+36)

1. Stufe:

(1RS,2RS,5SR)-2-Dimethylaminomethyl-1-(3-methoxy-phenyl)-5-methyl-cyclohexanol, Hydrochlorid (37)

95 ml (750 mmol) 1-Brom-3-methoxy-benzol wurden in 425 ml trockenem Tetrahydrofuran gelöst und auf -75° C gekühlt. Nach Zugabe von 750 mmol 1,6 molarer n-Butyllithiumlösung in Hexan wurde eine Stunde bei -75° C gerührt.

Anschließend wurden 82 g (484 mmol) (2RS,5SR)-2-Dimethylaminomethyl-5-methyl-cyclohexanon, hergestellt aus 3-Methylcyclohexanon, Dimethylaminhydrochlorid und Paraformaldehyd in Eisessig, gelöst in 120 ml trockenem Tetrahydrofuran, zugetropft. Innerhalb von 2,5 Stunden wurde das Reaktionsgemisch auf Raumtemperatur erwärmt.

Zur Aufarbeitung wurden unter Eisbadkühlung 200 ml Wasser zugetropft, so daß die Innentemperatur 15° C nicht überstieg. Nach Phasentrennung wurde die wäßrige Phase dreimal mit 50 ml Essigsäureethylester extrahiert. Die vereinigten organischen Phasen wurden über Natriumsulfat getrocknet. Nach destillativer Entfernung des Lösungsmittels wurde der Rückstand in 700 ml Aceton gelöst und mit Trimethylchlorsilan/Wasser versetzt. Bei 4 - 5° C kristallisierten 67 g (48 % der Theorie) Hydrochlorid (37) mit einem Schmelzpunkt von 173 - 175° C aus.

2. Stufe:

Enantiomere von (37):

(+)-(1R,2R,5S)-2-Dimethylaminomethyl-1-(3-methoxy-phenyl)-5-methyl-cyclohexanol, Hydrochlorid (+37) und

(-)-(1S,2S,5R)-2-Dimethylaminomethyl-1-(3-methoxy-phenyl)-5-methyl-cyclohexanol, Hydrochlorid (-37)

Aus (37) wurde mit Dichlormethan/Natronlauge die Base freigesetzt. Nach dem Trocknen der Lösung wurde Dichlormethan im Vakuum abdestilliert. Das Racemat wurde dann auf der chiralen HPLC-Säule getrennt. Aus den erhaltenen Enantiomeren wurden durch Umsetzung mit Trimethylchlorsilan/Wasser in 2-Butanon die Hydrochloride mit einem Schmelzpunkt von 151 - 153° C isoliert.

(+37): Ausbeute: 43 % der Theorie $[\alpha]_D^{RT}$ = +36,4° (c = 1,01; Methanol)

(-37): Ausbeute: 44 % der Theorie $[\alpha]_D^{RT}$ = -37,7° (c = 1,01; Methanol)

3. Stufe:

(-)-(1R,4S)-[2-(3-Methoxy-phenyl)-4-methyl-cyclohex-2-enylmethyl]-dimethylamin, Hydrochlorid (-38) und

(+)-(1S,4R)-[2-(3-Methoxy-phenyl)-4-methyl-cyclohex-2-enylmethyl]-dimethylamin, Hydrochlorid (+38)

Die Methoxyverbindungen (-37) und (+37) aus der 2. Stufe wurden unter den in Beispiel 5 angegebenen Bedingungen in die Hydrochloride (+38) und (-38) in einer Ausbeute von 87 % der Theorie und mit einem Schmelzpunkt von 122 - 123° C überführt.

4. Stufe:

(-)-(3S,6R)-3-(6-Dimethylaminomethyl-3-methyl-cyclohex-1-enyl)-phenol, Hydrochlorid (-36) und

(+)-(3R,6S)-3-(6-Dimethylaminomethyl-3-methyl-cyclohex-1-enyl)-phenol, Hydrochlorid (+36)

Aus den nach Stufe 3 erhaltenen Basen wurden unter den in Beispiel 2 angegebenen Bedingungen durch Umsetzung mit Diisobutylaluminiumhydrid und anschließender Hydrochloridfällung mit Trimethylchlorsilan/Wasser in 2-Butanon die Hydrochloride (-36) und (+36) in einer Ausbeute von 79 % der Theorie und einem Schmelzpunkt von 131 - 133° C erhalten.

(-36): $[\alpha]_D^{RT}$ = - 75,5° (c = 0,96; Methanol)

(+36): $[\alpha]_D^{RT}$ = + 77,7° (c = 1,08; Methanol)

**Beispiel 26 (nicht gegenstand der Ansprüche)**

(-)-(R)-3-(6-Dimethylaminomethyl-cyclohex-1-enyl)-phenol, Hydrochlorid (-39)

28,8 g (0,1 mol) (+)-(1R,2R)-3-(2-Dimethylaminomethyll-hydroxy-cyclohexyl)-phenol, Hydrochlorid wurden in 450 ml konzentrierter Ameisensäure gelöst und zwei Stunden unter Rückfluß erhitzt. Anschließend wurde die Ameisensäure im Wasserstrahlvakuum abdestilliert und aus dem Rückstand mit Dichlormethan/wäßriger Natriumcarbonatlösung die Base freigesetzt, aus der mit konzentrierter Salzsäure in Aceton 21,8 g (81,4 % der Theorie) Hydrochlorid (-39) mit einem Schmelzpunkt von 216 - 217° C erhalten wurden.

(-39): $[\alpha]_D^{RT}$ = -96,6° (c = 1,04; Methanol)

**Beispiel 27 (nicht gegenstand der Ansprüche)**

(+)-(S)-3-(6-Dimethylaminomethyl-cyclohex-1-enyl)-phenol, Hydrochlorid (+39)

Unter den in Beispiel 26 angegebenen Bedingungen wurden aus 28,8 g (0,1 mol) (-)-(1S,2S)-3-(2-Dimethylaminomethyl-1-hydroxy-cyclohexyl)-phenol, Hydrochlorid 21,8 g (81,4 % der Theorie) Hydrochlorid (+39) mit einem Schmelzpunkt von 216 - 217° C erhalten.

(+39): $[\alpha]_D^{RT}$ = +89,0° (c = 0,99; Methanol)

## Pharmakologische Untersuchungen

### Analgesieprüfung im Writhing-Test an der Maus

Die analgetische Wirksamkeit wurde im Phenylchinon-induzierten Writhing an der Maus, modifiziert nach I. C. Hendershot, J. Forsaith, J. Pharmacol. Exp. Ther. 125, 237 - 240 (1959) untersucht. Dazu wurden männliche NMRI-Mäuse mit einem Gewicht von 25 - 30 g eingesetzt. Gruppen von 10 Tieren pro Substanzdosis wurden 10 Minuten nach intravenöser Gabe einer erfindungsgemäßen Verbindung 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45° C) intraperitoneal appliziert. Die Tiere wurden einzeln in Beobachtungskäfige gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Aus der dosisabhängigen Abnahme der Writhingreaktionen im Vergleich zu parallel untersuchten Tiergruppen, denen keine erfindungsgemäße Verbindungen appliziert wurde, wurden mittels Regressionsanalyse (Auswerteprogramm Martens EDV Service, Eckental) die $ED_{50}$-Werte der Writhingreaktion berechnet.

Alle untersuchten erfindungsgemäßen Verbindungen zeigten eine ausgeprägte analgetische Wirkung, die im Vergleich zu Tramadol verstärkt war.

Die Ergebnisse sind in der nachfolgenden Tabelle zusammengefaßt.

Tabelle:

| Analgesieprüfung im Writhing-Test an der Maus | |
|---|---|
| Verbindung hergestellt nach Beispiel | $ED_{50}$ (mg/kg) |
| 2 | 1,37 |
| 3 (+)-Enantiomer | 2,25 |
| 3 (-)-Enantiomer | 0,98 |
| 4 | 1,64 |
| 12 | 0,97 |
| 13 | 2,96 |
| 15 | 1,33 |
| 18 | 2,07 |
| 20 (+)-Enantiomer (erfindungsgemäß) | 1,40 |
| 22 (-)-Enantiomer (erfindungsgemäß) | 2,12 |
| 24 | 1,35 |
| 25 (-)-Enantiomer | 0,90 |
| 26 (-)-Enantiomer | 1,04 |
| 27 (+)-Enantiomer | 1,60 |
| zum Vergleich: Tramadol | 3,68 |

**Patentansprüche**

1. 1-Dimethylaminomethyl-2-phenyl-cycloalk-2-enverbindungen der Formel I,

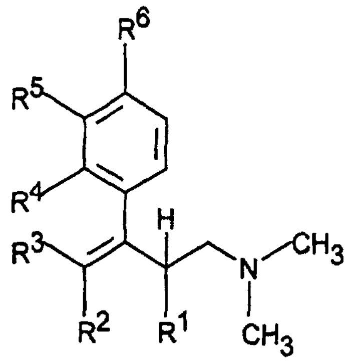

in der $R^1$ und $R^2$ zusammen-$(CH_2)_{2-4}$-, -$(CH_2)_2$-$CHR^7$ oder -$CH_2$-$CHR^7$-$CH_2$- darstellen,
$R^3$ H oder $C_{1-5}$-Alkyl bedeutet,
$R^4$ H, OH, $C_{1-4}$-Alkyl, O-$C_{1-4}$-Alkyl, O-Benzyl, $CF_3$, O-$CF_3$, Cl, F oder $OR^8$ bedeutet,
$R^5$ H darstellt und
$R^6$ H, OH, $C_{1-4}$-Alkyl, O-$C_{1-4}$-Alkyl, O-Benzyl, $CF_3$, O-$CF_3$, Cl, F oder $OR^8$ bedeutet,
mit der Maßgabe, daß zwei der Reste $R^4$, $R^5$ oder $R^6$ H sind,
$R^7$ $C_{1-8}$-Alkyl, $C_{3-8}$-Cycloalkyl, O-$C_{1-4}$-Alkyl, O-Benzyl, $CF_3$, Cl oder F bedeutet,
$R^8$ CO-$C_6H_4$-$R^{11}$ bedeutet,
$R^{11}$ OC(O)-$C_{1-3}$-Alkyl in ortho-Stellung oder $CH_2$-N-$(R^{15})_2$ in meta- oder para-Stellung, wobei $R^{15}$ $C_{1-4}$-Alkyl oder beide Reste $R^{15}$ zusammen mit N den 4-Morpholino-Rest bilden, bedeutet,
in Form ihrer Basen und/oder Salze von physiologisch verträglichen Säuren, als Enantiomere oder Racemate.

2. Verbindungen gemäß Anspruch 1, **dadurch gekennzeichnet, daß**
$R^1$ und $R^2$ zusammen -$(CH_2)_{2-4}$- oder -$(CH_2)_2$-$CHR^7$ bedeuten,
$R^3$ H oder $C_{1-3}$-Alkyl bedeutet,
$R^4$ H, OH, $CF_3$, Cl, F oder $OR^8$ bedeutet,
$R^5$ H darstellt und
$R^6$ H, OH, O-$C_{1-4}$-Alkyl, O-Benzyl, $CF_3$, Cl, F oder $OR^8$ bedeutet,
mit der Maßgabe, daß zwei der Reste $R^4$, $R^5$ oder $R^6$ H sind und
$R^7$ $C_{1-4}$-Alkyl, $CF_3$, Cl oder F bedeutet.

3. Verbindungen gemäß Anspruch 1 oder 2, **dadurch gekennzeichnet, daß**
$R^1$ und $R^2$ zusammen -$(CH_2)_{2-3}$- oder -$(CH_2)_2$-$CHR^7$ bedeuten,
$R^3$ H, $CH_3$ oder $CH_2CH_3$ bedeutet,
$R^4$ H oder OH, $R^5$ H und $R^6$ H, OH oder $CF_3$ bedeuten, mit der Maßgabe, daß zwei der Reste $R^4$, $R^5$ oder $R^6$ H sind.

4. Verfahren zur Herstellung einer 1-Dimethylaminomethyl-2-phenyl-cycloalk-2-enverbindung der Formel I,

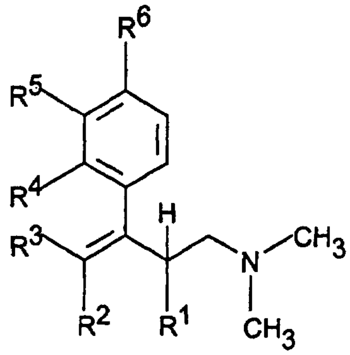

in der $R^1$ und $R^2$ zusammen $-(CH_2)_{2-4}-$, $-(CH_2)_2-CHR^7$ oder $-CH_2-CHR^7-CH_2-$ darstellen,
$R^3$ H oder $C_{1-5}$-Alkyl bedeutet,
$R^4$ H, $C_{1-4}$-Alkyl, O-$C_{1-4}$-Alkyl, O-Benzyl, $CF_3$, O-$CF_3$, Cl oder F bedeutet,
$R^5$ H bedeutet, und
$R_6$ H, $C_{1-4}$-Alkyl, O-$C_{1-4}$-Alkyl, O-Benzyl, $CF_3$, O-$CF_3$, Cl oder F bedeutet,
mit der Maßgabe, daß zwei der Reste $R^4$, $R^5$ oder $R^6$ H sind,
$R^7$ $C_{1-8}$-Alkyl, $C_{3-8}$-Cycloalkyl, O-$C_{1-4}$-Alkyl, O-Benzyl, $CF_3$, Cl oder F bedeutet, **dadurch gekennzeichnet, daß** man ein β-Dimethylaminoketon der Formel II

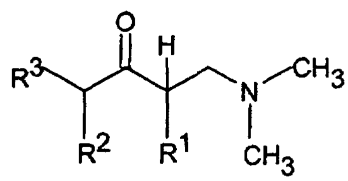

mit einer metallorganischen Verbindung der Formel III

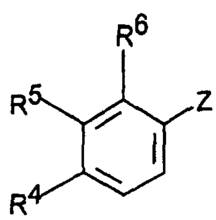

in der Z MgCl, MgBr, MgI oder Li bedeutet, zu einem tertiären Alkohol der Formel IV

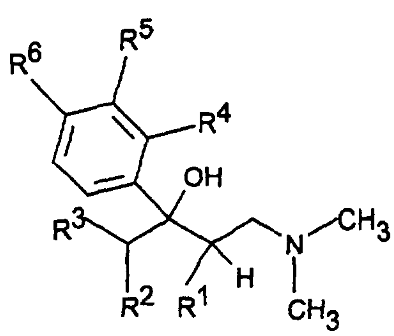

umsetzt, welcher anschließend zu einer Verbindung der Formel I dehydratisiert wird.

**5.** Verfahren zur Herstellung einer 1-Dimethylaminomethyl-2-phenyl-cycloalk-2-enverbindung der Formel I,

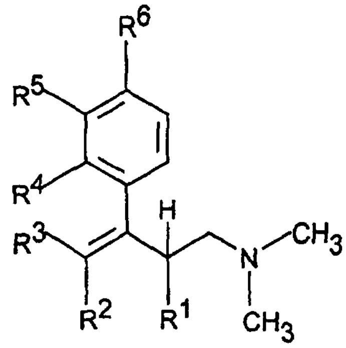

in der $R^1$ und $R^2$ zusammen $-(CH_2)_{2-4}-$, $-(CH_2)_2-CHR^7$ oder $-CH_2-CHR^7-CH_2-$ bedeuten,
$R^3$ H oder $C_{1-5}$-Alkyl bedeutet,
einer der Reste $R^4$ oder $R^6$ OH bedeutet und zwei der Reste $R^4$, $R^5$ oder $R^6$ H sind,
$R^7$ $C_{1-8}$-Alkyl, $C_{3-8}$-Cycloalkyl, O-$C_{1-4}$-Alkyl, O-Benzyl, $CF_3$, Cl oder F bedeutet,
**dadurch gekennzeichnet, daß** man eine Verbindung der Formel I, in der einer der Reste $R^4$ oder $R^6$ O-$CH_3$ bedeutet und zwei der Reste $R^4$, $R^5$ oder $R^6$ H sind, mit Diisobutylaluminiumhydrid umsetzt oder eine Verbindung der Formel I, in der einer der Reste $R^4$ oder $R^6$ O-Benzyl bedeutet und zwei der Reste $R^4$, $R^5$ oder $R^6$ H sind, reduktiv debenzyliert.

**6.** Arzneimittel enthaltend als pharmazeutischen Wirkstoff wenigstens eine 1-Dimethylamenomethyl-2-phenyl-cycloalk-2-enverbindung der Formel I gemäß Anspruch 1 in Form ihrer Base und/oder ihres Salzes einer physiologisch verträglichen Säure, als Enantiomere oder Racemat.

**7.** Arzneimittel gemäß Anspruch 6, **dadurch gekennzeichnet, daß** es ein Schmerzmittel ist.

**8.** Verwendung wenigstens einer 1-Dimethylaminomethyl-2-phenyl-cycloalk-2-enverbindung der Formel I gemäß Anspruch 1 in Form ihrer Base und/oder ihres Salzes einer physiologisch verträglichen Säure, als Enantiomere oder Racemat zur Herstellung eines Arzneimittels.

**9.** Verwendung gemäß Anspruch 8, **dadurch gekennzeichnet, daß** das Arzneimittel ein Schmerzmittel ist.

## Claims

**1.** 1-dimethylaminomethyl-2-phenyl-cycloalk-2-ene compounds of formula I

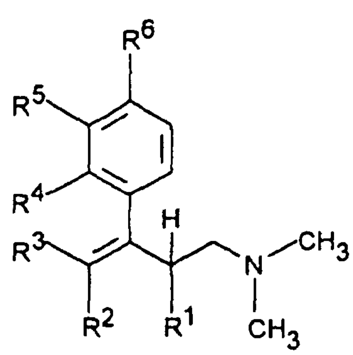

wherein $R^1$ and $R^2$ together represent $-(CH_2)_{2-4}-$, $-(CH_2)_2-CHR^7$ or $-CH_2-CHR^7-CH_2-$,
$R^3$ denotes H or $C_{1-5}$ alkyl,
$R^4$ denotes H, OH, $C_{1-4}$ alkyl, O-$C_{1-4}$ alkyl, O-benzyl, $CF_3$, O-$CF_3$, Cl, F or $OR^8$,

$R^5$ represents H, and
$R^6$ denotes H, OH, $C_{1-4}$ alkyl, O-$C_{1-4}$ alkyl, O-benzyl, $CF_3$, O-$CF_3$, Cl, F or $OR^8$,
with the proviso that two of the radicals $R^4$, $R^5$ or $R^6$ are H,
$R^7$ denotes $C_{1-8}$ alkyl, $C_{3-8}$ cycloalkyl, O-$C_{1-4}$ alkyl, O-benzyl, $CF_3$, Cl or F,
$R^8$ denotes CO-$C_6H_4$-$R^{11}$,
$R^{11}$ denotes OC(O)-$C_{1-3}$ alkyl in the ortho position or $CH_2$-N-$(R^{15})_2$ in the meta or para position, wherein $R^{15}$ denotes $C_{1-4}$ alkyl or both radicals $R^{15}$ form the 4-morpholino radical together with N,
in the form of their bases and/or salts of physiologically compatible acids, as enantiomers or racemates.

**2.** Compounds according to claim 1, **characterised in that**
$R^1$ and $R^2$ together represent -$(CH_2)_{2-4}$-, or -$(CH_2)_2$-$CHR^7$,
$R^3$ denotes H or $C_{1-3}$ alkyl,
$R^4$ denotes H, OH, $CF_3$, Cl, F or $OR^8$,
$R^5$ represents H, and
$R^6$ denotes H, OH, O-$C_{1-4}$ alkyl, O-benzyl, $CF_3$, Cl, F or $OR^8$,
with the proviso that two of the radicals $R^4$, $R^5$ or $R^6$ are H, and
$R^7$ denotes $C_{1-4}$ alkyl, $CF_3$, Cl or F.

**3.** Compounds according to claims 1 or 2, **characterised in that**
$R^1$ and $R^2$ together denote -$(CH_2)_{2-3}$- or -$(CH_2)_2$-$CHR^7$,
$R^3$ denotes H, $CH_3$ or $CH_2CH_3$,
$R^4$ denotes H or OH, $R^5$ denotes H, and $R^6$ denotes H, OH or $CF_3$, with the proviso that two of the radicals $R^4$, $R^5$ or $R^6$ are H.

**4.** A method of preparing a 1-dimethylaminomethyl-2-phenyl-cycloalk-2-ene compound of formula I

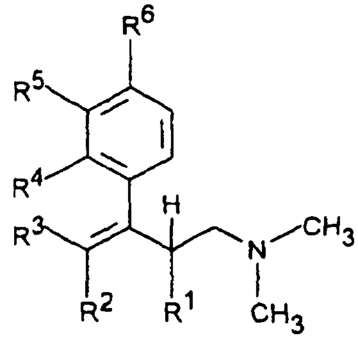

wherein $R^1$ and $R^2$ together represent -$(CH_2)_{2-4}$-, -$(CH_2)_2$-$CHR^7$ or -$CH_2$-$CHR^7$-$CH_2$-,
$R^3$ denotes H or $C_{1-5}$ alkyl,
$R^4$ denotes H, $C_{1-4}$ alkyl, O-benzyl, $CF_3$, O-$CF_3$, Cl or F,
$R^5$ represents H, and
$R^6$ denotes H, $C_{1-4}$ alkyl, O-$C_{1-4}$ alkyl, O-benzyl, $CF_3$, O-$CF_3$, Cl or F,
with the proviso that two of the radicals $R^4$, $R^5$ or $R^6$ are H,
$R^7$ denotes $C_{1-8}$ alkyl, C $_{3-8}$cycloalkyl, O-$C_{1-4}$alkyl, O-benzyl, $CF_3$ Cl or F, **characterised in that** a β-dimethylaminoketone of formula II

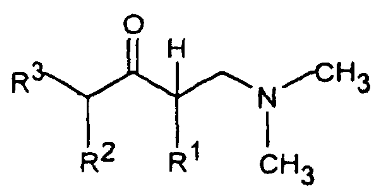

is reacted with an organometallic compound of formula III

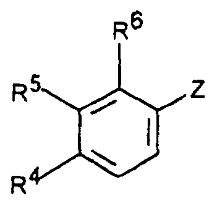

wherein Z denotes MgCl, MgBr, MgI or Li, to form a tertiary alcohol of formula IV

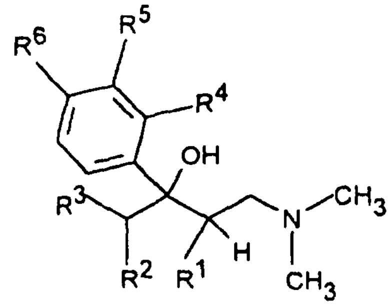

which is subsequently dehydrated to form a compound of formula I.

5. A method of preparing a 1-dimethylaminomethyl-2-phenyl-cycloalk-2-ene compound of formula I

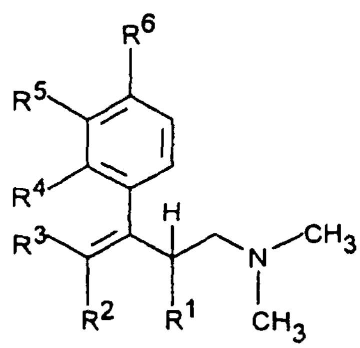

wherein $R^1$ and $R^2$ together represent $-(CH_2)_{2-4}-$, $-(CH_2)_2-CHR^7$ or $-CH_2-CHR^7-CH_2-$,
$R^3$ denotes H or $C_{1-5}$ alkyl,
one of the radicals $R^4$ or $R^6$ denotes OH and two of the radicals $R^4$, $R^5$ or $R^6$are H,
$R^7$ denotes $C_{1-8}$ alkyl, $C_{3-8}$ cycloalkyl, O-$C_{1-4}$ alkyl, O-benzyl, $CF_3$, Cl or F,
**characterised in that** a compound of formula I, in which one of the radicals $R^4$ or $R^6$ denotes O-$CH_3$ and two of the radicals $R^4$, $R^5$ or $R^6$ are H, is reacted with diisobutylaluminium hydride, or a compound of formula I, in which one of the radicals $R^4$ or $R^6$ denotes O-benzyl and two of the radicals $R^4$, $R^5$ or $R^6$ are H, is reductively debenzylated.

6. A drug, containing, as a pharmaceutical active ingredient, at least one 1-dimethylaminomethyl-2-phenyl-cycloalk-2-ene compound of formula I according to claim 1 in the form of its base and/or of its salt of a physiologically compatible acid, as enantiomers or as a racemate.

7. A drug according to claim 6, characteriseed in that it is a pain-killing drug.

8. Use of at least one 1-dimethylaminomethyl-2-phenyl-cycloalk-2-ene compound of formula I according to claim 1, in the form of its base and/or of its salt of a physiologically compatible acid, as enantiomers or as a racemate, for the production of a drug.

**9.** A use according to claim 8, **characterised in that** the drug is a pain-killing drug.

## Revendications

**1.** Composés de 1-diméthylaminométhyl-2-phényl-cycloalc-2-ènes de formule I

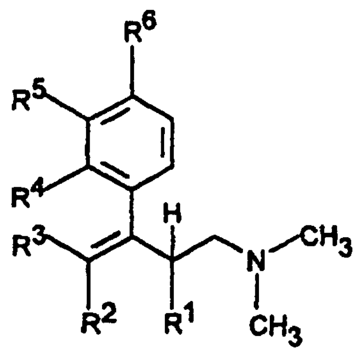

dans laquelle $R^1$ et $R^2$ représentent ensemble un groupe - $(CH_2)_{2-4}$-, - $(CH_2)_2$-$CHR^7$ ou -$CH_2$-$CHR^7$-$CH_2$-,
$R^3$ représente H ou un groupe alkyle en $C_1$ à $C_5$,
$R^4$ représente H, un groupe OH, alkyle en $C_1$ à $C_4$, O-(alkyle en $C_1$ à $C_4$), O-benzyle, $CP_3$, O-$CF_3$, Cl, F ou $OR^8$,
$R^5$ représente H et
$R^6$ représente H, un groupe OH, alkyle en $C_1$ à $C_4$, O-(alkyle en $C_1$ à $C_4$), O-benzyle, $CF_3$, O-$CF_3$, Cl, F ou $OR^8$,
sous réserve que deux des restes $R^4$, $R^5$ ou $R^6$ représentent H,
$R^7$ est un groupe alkyle en $C_1$ à $O_8$, cycloalkyle en $C_3$ à $C_8$, O-(alkyle en $C_1$ à $C_4$), O-benzyle, $CF_3$, Cl ou F,
$R^8$ est un groupe CO-$C_6H_4$-$R^{11}$,
$R^{11}$ est un groupe OC(O)-alkyle en $C_1$ à $C_3$ en position ortho ou un groupe $CH_2$-N-$(R^{15})_2$ en position méta ou para, $R^{15}$ représentant un groupe alkyle en $C_1$ à $C_4$, ou bien les deux restes $R^{15}$ forment conjointement avec N le reste 4-morpholino,
sous forme de leurs bases et/ou de leurs sels d'acides acceptables du point de vue physiologique, comme énantiomères ou comme racémates.

**2.** Composés suivant la revendication 1, **caractérisés en ce que**
$R^1$ et $R^2$ forment ensemble un groupe -$(CH_2)_{2-4}$- ou -$(CH_2)_2$-$CHR^7$,
$R^3$ représente H ou un groupe alkyle en $C_1$ à $C_3$,
$R^4$ représente H, un groupe OH, $CF_3$, Cl, F ou $OR^8$,
$R^5$ représente H et
$R^6$ représente H, un groupe OH, O-(alkyle en $C_1$ à $C_4$), O-benzyle, $CF_3$, Cl, F ou $OR^8$,
sous réserve que deux des restes $R^4$, $R^5$ ou $R^6$ représentent H et
$R^7$ est un groupe alkyle en $C_1$ à $C_4$, $CF_3$, Cl ou F.

**3.** Composés suivant la revendication 1 ou 2, **caractérisés en ce que**
$R^1$ et $R^2$ forment ensemble un groupe -$(CH_2)_{2-3}$- ou -$(CH_2)_2$-$CHR^7$,
$R^3$ représente H, un groupe $CH_3$ ou $CH_2CH_3$,
$R^4$ représente H ou OH, $R^5$ représente H et $R^6$ représente H, OH ou $CF_3$, sous réserve que deux des restes $R^4$, $R^5$ ou $R^6$ représentent H.

**4.** Procédé de production d'un composé de 1-diméthylaminométhyl-2-phényl-cycloalc-2-ène de formule I

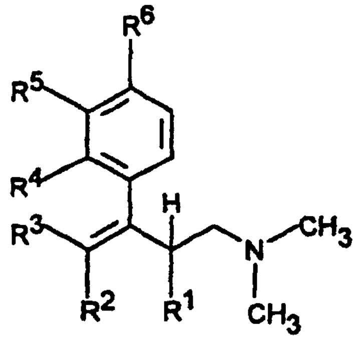

dans laquelle $R^1$ et $R^2$ forment ensemble un groupe $-(CH_2)_{2-4}-$, $-(CH_2)_2-CHR^7$ ou $-(CH_2)-CHR^7-CH_2-$, $R^3$ représente H ou un groupe alkyle en $C_1$ à $C_5$, $R^4$ représente H, un groupe alkyle en $C_1$ à $C_4$, O-(alkyle en $C_1$ à $C_4$), O-benzyle, $CF_3$, $O-CF_3$, Cl ou F, $R^5$ représente H et $R^6$ représente H, un groupe alkyle en $C_1$ à $C_4$, O-(alkyle en $C_1$ à $C_4$), O-benzyle, $CF_3$, $O-CF_3$, Cl ou F, sous réserve que deux des restes $R^4$, $R^5$ ou $R^6$ représentent H, $R^7$ est un groupe alkyle en $C_1$ à $C_8$, cycloalkyle en $C_3$ à $C_8$, O-(alkyle en $C_1$ à $C_4$), O-benzyle, $CF_3$, Cl ou F, **caractérisé en ce qu'**on fait réagir une β-diméthylaminocétone de formule II

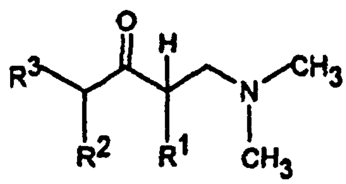

avec un composé organométallique de formule III

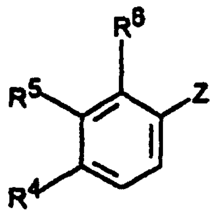

dans laquelle Z représente MgCl, MgBr, MgI ou Li, pour former un alcool tertiaire de formule IV

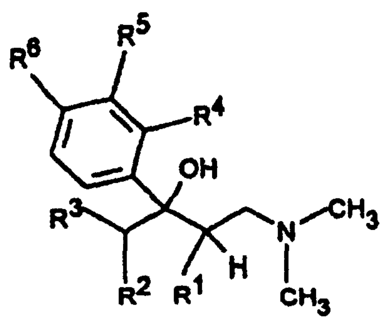

qu'on déshydrate ensuite en un composé de formule I.

**5.** Procédé de production d'un composé de 1-diméthylaminométhyl-2-phényl-cycloalc-2-ène de formule I

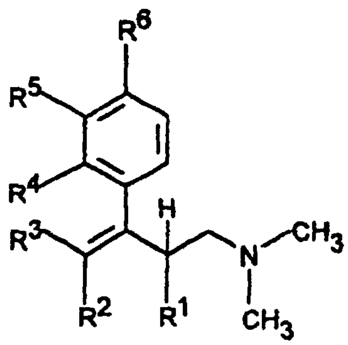

dans laquelle $R^1$ et $R^2$ forment ensemble un groupe $(CH_2)_{2-4}$-, -$(CH_2)_2$-$CHR^7$ ou - $(CH_2)$-$CHR^7$-$CH_2$-,
$R^3$ représente H ou un groupe alkyle en $C_1$ à $C_5$,
l'un des restes $R^4$ ou $R^6$ représente un groupe OH et deux des restes $R^4$, $R^5$ ou $R^6$ représentent H,
$R^7$ est un groupe alkyle en $C_1$ à $C_8$, cycloalkyle en $C_3$ à $C_8$, O-(alkyle en $C_1$ à $C_4$), O-benzyle, $CF_3$, Cl ou F,
**caractérisé en ce qu'**on fait réagir un composé de formule I dans laquelle l'un des restes $R^4$ ou $R^6$ représente O-$CH_3$ et deux des restes $R^4$, $R^5$ ou $R^6$ représentent H, avec l'hydrure de diisobutylaluminium ou bien on débenzyle par voie de réduction un composé de formule I dans laquelle l'un des restes $R^4$ ou $R^6$ est un groupe O-benzyle et deux des restes $R^4$, $R^5$ ou $R^6$ représentent H.

**6.** Médicament contenant comme substance active pharmaceutique au moins un composé de 1-diméthylaminométhyl-2-phényl-cycloalc-2-ène de formule I suivant la revendication 1 sous forme de sa base et/ou de son sel avec un acide acceptable du point de vue physiologique, comme énantiomère ou racémate.

**7.** Médicament suivant la revendication 6, **caractérisé en ce qu'**il est un analgésique.

**8.** Utilisation d'au moins un composé de 1-diméthylaminométhyl-2-phényl-cycloalc-2-ène de formule I suivant la revendication 1 sous forme de sa base et/ou de son sel d'acide acceptable du point de vue physiologique, comme énantiomère ou comme racémate pour la préparation d'un médicament.

**9.** Utilisation suivant la revendication 8, **caractérisée en ce que** le médicament est un analgésique.